(19) Europäisches Patentamt
European Patent Office
Office européen des brevets

(11) EP 1 379 138 B1

(12) **EUROPÄISCHE PATENTSCHRIFT**

(45) Veröffentlichungstag und Bekanntmachung des Hinweises auf die Patenterteilung:
**16.11.2005 Patentblatt 2005/46**

(21) Anmeldenummer: **02766612.2**

(22) Anmeldetag: **02.04.2002**

(51) Int Cl.[7]: **A01N 51/00**
// (A01N51/00, 53:08),
A01N25:02

(86) Internationale Anmeldenummer:
**PCT/EP2002/003619**

(87) Internationale Veröffentlichungsnummer:
**WO 2002/087338 (07.11.2002 Gazette 2002/45)**

(54) **DERMAL APPLIZIERBARE FLÜSSIGE FORMULIERUNGEN ZUR BEKÄMPFUNG VON PARASITIERENDEN INSEKTEN AN TIEREN**

LIQUID FORMULATIONS FOR DERMAL APPLICATION IN TREATMENT OF PARASITIC INSECTS IN ANIMALS

FORMULATIONS LIQUIDES A APPLICATION CUTANEE POUR LUTTER CONTRE DES INSECTES PARASITES SUR DES ANIMAUX

(84) Benannte Vertragsstaaten:
**AT BE CH CY DE DK ES FI FR GB GR IE IT LI LU MC NL PT SE TR**
Benannte Erstreckungsstaaten:
**SI**

(30) Priorität: **09.04.2001 DE 10117676**

(43) Veröffentlichungstag der Anmeldung:
**14.01.2004 Patentblatt 2004/03**

(73) Patentinhaber: **Bayer HealthCare AG
51368 Leverkusen (DE)**

(72) Erfinder:
- **SIRINYAN, Kirkor
51467 Bergisch Gladbach (DE)**
- **DORN, Hubert
42115 Wuppertal (DE)**
- **GILGES, Martin
50667 Köln (DE)**
- **HANSEN, Olaf
42799 Leichlingen (DE)**

(56) Entgegenhaltungen:
**EP-A- 0 682 869**
**WO-A-96/17520**
**WO-A-02/43494**
**FR-A- 2 784 011**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue, hautverträgliche, dermal applizierbare flüssige Formulierungen enthaltend Permethrin und Agonisten oder Antagonisten der nicotinergen Acetylcholinrezeptoren von Insekten zur Bekämpfung von parasitierenden Insekten an Tieren.

Die Verwendung von topischen Formulierungen enthaltend Permethrin, (3-phenoxy phenyl) Methyl 3-(2,2-dichloroethenyl)-2,2-dimethylcyclopropanecarhoxylate, (CAS No [52645-53-1] zur Bekämpfung von parasitierenden Insekten an Tieren ist bekannt (vgl. z.B. WO 95/17 090, JP-07 247 203, EP-A-567 368, EP-A-461 962, US-5 236 954 und US-5 074 252).

Agonisten oder Antanogisten der nicotinergen Acetylcholinrezeptoren von Insekten sind bekannt, z.B. aus Agonisten oder Antanogisten der nicotinergen Acetylcholinrezeptoren von Insekten sind bekannt z.B. aus Europäische Offenlegungsschriften Nr. 464 830, 428 941, 425 978, 386 565, 383 091, 375 907, 364 844, 315 826, 259 738, 254 859, 235 725, 212 600, 192 060, 163 855, 154 178, 136 636, 303 570, 302 833, 306 696, 189 972, 455 000, 135 956, 471 372, 302 389; Deutsche Offenlegungsschriften Nr. 3 639 877, 3 712 307; Japanische Offenlegungsschriften Nr. 03 220 176, 02 207 083, 63 307 857, 63 287 764, 03 246 283, 04 9371, 03 279 359, 03 255 072; US-Patentschriften Nr. 5 034 524, 4 948 798, 4 918 086, 5 039 686, 5 034 404; PCT-Anmeldungen Nr. WO 91/17 659, 92/4965; Französische Anmeldung Nr. 2 611 114; Brasilianische Anmeldung Nr. 88 03 621. Die Verwendung von Spot-on-Formulierungen enthaltend Agonisten oder Antagonisten der nicotinergen Acetylcholinrezeptoren von Insekten zur Bekämpfung von parasitierenden Insekten an Tieren ist ebenso bekannt (siehe beispielsweise WO 98/27 817, EP-A-682 869 und EP 0 976 328).

Im Stand der Technik wurden auch bereits Kombinationen von Permethrin mit Agonisten oder Antanogisten der nicotinergenen Acetylcholinrezeptoren von Insekten zur Bekämpfung von Parasiten beschrieben (vgl. z.B. CN-1 245 637, WO 00/54 591, US-6 080 796, EP-A-981 955, US-6033731, JP-07 089 803). WO 02/43494 offenbart Mittel mit erhöhter akarizider Wirkung, es werden Pyrethroide und Nicotinyl-Verbindungen in Kombination eingesetzt.

Der Nachteil der Spot-on-Formulierungen auf Permethrin-Basis liegt in der geringen Wirksamkeit gegen Zecken und Flöhen.

Spot-on-Formulierungen auf Basis von Agonisten oder Antagonisten der nicotinergen Acetylcholinrezeptoren weisen in der Regel sehr gute Flohwirksamkeit auf. Sie haben jedoch den Nachteil, dass sie gegen Zecken unwirksam sind.

Die bislang bekannten Kombinationsformulierungen enthaltend Permethrin und Agonisten oder Antagonisten der nicotinergen Acetylcholinrezeptoren sind leider zur Bekämpfung von Parasiten an Tieren, insbesondere Kleintieren, nicht gut geeignet. Sie erfordern den Einsatz von größeren Wirkstoffmengen und führen in vielen Fällen zu Hautirritationen. Permethrin ist eine stark aprotische Verbindung während Agonisten und Antagonisten der nicotinergen Acetylcholinrezeptoren, insbesondere die Imidaclopridanaloga, protische Verbindungen sind. Es ist daher nicht einfach, eine dermal applizierbare flüssige Formulierung zu finden, die beide Wirkstoffe enthält und folgende Eigenschaften aufweist:

- gute Löslichkeit der Wirkstoffe
- gute Hautverträglichkeit
- geringe Toxizität
- geringe Hautpenetration (da die Wirkstoffe vorzugsweise nicht-systemisch wirken sollen)
- hohe Wirksamkeit.

Aus diesem Grunde war bislang für eine erfolgreiche Zecken- und Flohbekämpfung eine Doppelbehandlung der Tiere mit den besagten Spot-on-Formulierungen erforderlich. Aus ökologischen und ökonomischen Gründen ist es wünschenswert, diese Formulierungen durch solche zu ersetzen, die gut hautverträglich sowie toxikologisch unbedenklich sind und sich ferner sich bei einem kleinen Applikationsvolumen z.B. (0,1 ml / 1.0 kg [Körpergewicht des zu behandelnden Tieres]) durch ihre gute Langzeitwirkung von mindestens drei bis vier Wochen, vor allem gegen Zecken und Flöhe, auszeichnen. Weiterhin sollte eine solche Formulierung in allen Klimazonen eine ausreichende Lagerungsstabilität aufweisen, üblicherweise mindestens drei Jahre z.B. in den herkömmlichen Spot-on-Tuben.

Aufgabe der vorliegenden Erfindung war daher die Bereitstellung einer haut- und umweltverträglichen, anwenderfreundlichen, gegen parasitierende Insekten, insbesondere gegen Zecken und Flöhe, wirksamen Formulierung für die dermale Applikation enthaltend Permethrin und Agonisten oder Antanogisten der nicotinergen Acetylcholinrezeptoren von Insekten.

Diese Aufgabe wird durch die im folgenden beschriebenen erfindungsgemäßen Mittel gelöst.

Die vorliegende Erfindung betrifft

1. Mittel enthaltend,

a) 35 - 60 Gew.-% des Wirkstoffs Permethrin
b) 2.5 - 12.5 Gew.-% Imidacloprid oder Imidaclopridanalogon der Formel (I):

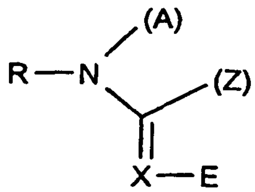

(I),

in welcher

R für Wasserstoff, gegebenenfalls substituierte Reste der Gruppe Acyl, Alkyl, Aryl, Aralkyl, Heteroaryl oder Heteroarylalkyl steht;

A für eine monofunktionelle Gruppe aus der Reihe Wasserstoff, Acyl, Alkyl, Aryl steht oder für eine bifunktionelle Gruppe steht, die mit dem Rest Z verknüpft ist;

E für einen elektronenziehenden Rest steht;

X für die Reste -CH= oder =N- steht, wobei der Rest -CH= anstelle eines H-Atoms mit dem Rest Z verknüpft sein kann;

Z für eine monofunktionelle Gruppe aus der Reihe Alkyl, -O-R, -S-R,

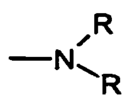

steht, oder für eine bifunktionelle Gruppe steht, die mit dem Rest A oder dem Rest X verknüpft ist,
wobei die Substituenten der oben aufgeführten gegebenenfalls substituierten Reste ausgewählt sind aus: Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen, wobei die Halogenatome gleich oder verschieden sind; Hydroxy; Halogen; Cyano; Nitro; Amino; Monoalkyl- und Dialkylamino mit 1 bis 4 Kohlenstoffatomen, Carboxyl; Carbalkoxy mit 2 bis 4; Sulfo (-$SO_3H$); Alkylsulfonyl mit 1 bis 4 Kohlenstoffatomen; Arylsulfonyl mit 6 oder 10 Arylkohlenstoffatomen sowie Heteroarylamino und Heteroarylalkylamino;
c) 27.5 - 62.5 Gew.-% N-Methylpyrrolidon
d) 0 - 5 Gew.-% Wasser
e) 0 - 0,5 Gew.-% phenolische Antioxidantien und
f) 0 - 0,5 Gew.-% organische Säuren.

Die Angaben in Gewichtsprozent beziehen sich auf das Gesamtgewicht.

Gemäß einer bevorzugten Ausführungsform enthalten die erfindungsgemäßen Mittel zusätzlich:

g) 2,5 - 10 Gew.-% Co-Lösungsmittel.

Die erfindungsgemäßen Mittel sind üblicherweise flüssig und eignen sich für die dermale Applikation, insbesondere als sogenannte Pour-on- oder Spot-on-Formulierungen.

Die ektoparasitizide Wirksamkeit der erfindungsgemäßen Mittel enthaltend Permethrin in Kombination mit Imidacloprid oder einem Imidacloprid-Analogon liegt überraschenderweise höher als von den Wirkungen der Einzelkomponenten zu erwarten war. Durch Anwendung dieser Mittel können daher die Aufwandmengen an Wirkstoff reduziert sowie die Langzeitwirkung erhöht werden. Ihre Anwendung bringt demzufolge ökonomische und ökologische Vorteile.

Die erfindungsgemäßen Mittel eignen sich hervorragend für den Einsatz bei der Parasitenbekämpfung.

Als Parasiten seien genannt:

Aus der Ordnung der Anoplura z.B. Haematopinus spp., Linognathus spp., Solenopotes spp., Pediculus spp., Pthirus spp.;
aus der Ordnung der Mallophaga z.B. Trimenopon spp., Menopon spp., Eomenacanthus spp., Menacanthus spp., Trichodectes spp., Felicola spp., Damalinea spp., Bovicola spp;
aus der Ordnung der Diptera z.B. Aedes spp., Culex spp., Simulium spp., Phlebotomus spp., Chrysops spp., Tabanus spp., Musca spp., Hydrotaea spp., Muscina spp., Haemato-bosca spp., Haematobia spp., Stomoxys spp., Fannia spp., Glossina spp., Lucilia spp., Calliphora spp., Auchmero-myia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippo-bosca spp..

Aus der Ordnung der Siphonaptera z.B. Ctenocephalides spp., Echidnophaga spp., Ceratophyllus spp., Pulex spp.
Aus der Ordnung der Metastigmata z.B. Hyalomma spp., Rhipicephalus spp., Boophilus spp., Amblyomma spp., Haemaphysalis spp., Dermacentor spp., Ixodes spp., Argas spp., Ornithodorus spp., Otobius spp.;
aus der Ordnung der Mesostigmata z.B. Dermanyssus spp., Ornithonyssus spp., Pneumonyssus spp..
Aus der Ordnung der Prostigmata z.B. Cheyletiella spp., Psorergates spp., Myobia spp., Demodex spp., Neotrombicula spp.;
aus der Ordnung der Astigmata z.B. Acarus spp., Myocop-tes spp., Psoroptes spp., Chorioptes spp., Otodectes spp., Sarcoptes spp., Notoedres spp., Knemidocoptes spp., Neoknemidocoptes spp. Cytodites spp., Lamino-sioptes spp..

Die erfindungsgemäßen Mittel eignen sich insbesondere bei der Bekämpfung von Ektoparasiten, vorzugsweise von Zecken und/oder Flöhen, an Tieren, insbesondere warmblütigen Tieren. Bevorzugt werden die erfindungsgemäßen Mittel bei Kleintieren eingesetzt. Unter Kleintieren werden hier insbesondere Hunde, Katzen und andere warmblütige Tiere, die nicht größer als Hunde sind, verstanden; d.h. sie haben ein Körpergewicht von üblicherweise nicht mehr als 90 kg, vorzugsweise nicht mehr als 50 kg. Besonders bevorzugt wurden die erfindungsgemäßen Mittel bei Hunden und Katzen, insbesondere bei Hunden, eingesetzt.

Da die behandelten Tiere in der Regel auch eine gewisse Menge des eingesetzten Mittels in der Umgebung verteilen, z. B. durch Reibung oder mit Debris, tritt die Wirkung der erfindungsgemäßen Mittel gegebenenfalls nicht nur direkt am Tier sondern auch in entsprechendem Maße in deren Umgebung ein.

Zur Herstellung der erfindungsgemäßen Flüssigformulierungen können alle üblichen Isomerenmischungen des Permethrin-Wirkstoffes eingesetzt werden. Die bevorzugte Isomerenmischung besteht aus 35 - 45 % cis- sowie 55 - 65 % trans-Permethrin. Die besonders bevorzugte Isomerenmischung besteht aus 37,5 - 42,5 % cis- sowie 57,5 - 62,5 % trans-Permethrin.

Die Permethrin-Mengen im erfindungsgemäßen Mittel können zwischen 35 - 60 % breit variiert werden. Bevorzugt werden Mengen im Bereich 45 - 60 %, besonders bevorzugt enthält das erfindungsgemäße Mittel Permethrin im Bereich 47.5 - 55 %.

Die Mengen an Imidacloprid oder Imidacloprid-Analogon können zwischen 2.5 - 12.5 % ebenso breit variiert werden, wobei Mengen im Bereich 5.0-10.0 % zu bevorzugen sind. Besonders bevorzugt wird Imidacloprid oder das Imidacloprid-Analogon in den erfindungsgemäßen Mitteln in Mengen im Bereich 7,5 - 10 % eingesetzt.

Selbstverständlich können die besagten Formulierungen noch weitere geeignete Wirkstoffe enthalten.

Als Beispiele seien wachstumshemmende Wirkstoffe und Synergisten genannt Pyriproxyfen {2-[1-methyl-2-(4-phenoxyphenoxy)-ethoxy]-pyridine CAS Nr.: 95737-68-1}, Methopren [(E,E)-1-methylethyl 11-methoxy-3,7,11-trimethyl-2,4-dodecadienoate CAS Nr.: 40596-69-8] und Triflumuron {2-chloro-N-[[[4-(trifluoro-methoxy)phenyl]amino] carbonyl]benzamide CAS Nr.: 64628-44-0}.

Als Agonisten oder Antanogisten der nicotinergen Acetylcholinrezeptoren von Insekten seien vorzugsweise die Imidaclopridanaloga genannt.

Unter Imidaclopridanaloga sollen Verbindungen der Formel (I) verstanden werden:

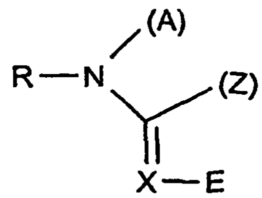

(I),

in welcher

R für Wasserstoff, gegebenenfalls substituierte Reste der Gruppe Acyl, Alkyl, Aryl, Aralkyl, Heteroaryl oder Heteroarylalkyl steht;

A für eine monofunktionelle Gruppe aus der Reihe Wasserstoff, Acyl, Alkyl, Aryl steht oder für eine bifunktionelle Gruppe steht, die mit dem Rest Z verknüpft ist;

E für einen elektronenziehenden Rest steht;

X für die Reste -CH= oder =N- steht, wobei der Rest -CH= anstelle eines H-Atoms mit dem Rest Z verknüpft sein kann;

Z für eine monofunktionelle Gruppe aus der Reihe Alkyl, -O-R, -S-R,

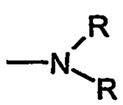

steht, oder für eine bifunktionelle Gruppe steht, die mit dem Rest A oder dem Rest X verknüpft ist.

Besonders bevorzugt sind Verbindungen der Formel (I), in welcher die Reste folgende Bedeutung haben:

R steht für Wasserstoff sowie für gegebenenfalls substituierte Reste aus der Reihe Acyl, Alkyl, Aryl, Aralkyl, Heteroaryl, Heteroarylalkyl.

Als Acylreste seien genannt Formyl, Alkylcarbonyl, Arylcarbonyl, Alkylsulfonyl, Arylsulfonyl, (Alkyl-)-(Aryl-)-phosphoryl, die ihrerseits substituiert sein können.

Als Alkyl seien genannt $C_{1-10}$-Alkyl, insbesondere $C_{1-4}$-Alkyl, im einzelnen Methyl, Ethyl, i-Propyl, sec.- oder t.-Butyl, die ihrerseits substituiert sein können.

Als Aryl seien genannt Phenyl, Naphthyl, insbesondere Phenyl.

Als Aralkyl seien genannt Phenylmethyl, Phenethyl.

Als Heteroaryl seien genannt Heteroaryl mit bis zu 10 Ringatomen und N, O, S insbesondere N als Heteroatomen. Im einzelnen seien genannt Thienyl, Furyl, Thiazolyl, Imidazolyl, Pyridyl, Benzthiazolyl.

Als Heteroarylalkyl seien genannt Heteroarylmethyl, Heteroarylethyl mit bis zu 6 Ringatomen und N, O, S, insbesondere N als Heteroatomen.

Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, Brom und Jod, insbesondere Fluor, Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Carboxyl; Carbalkoxy mit vorzugsweise 2 bis 4, insbesondere 2 oder 3 Kohlenstoffatomen, wie Carbo-

methoxy und Carboethoxy; Sulfo (-$SO_3H$); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl sowie Heteroarylamino und Heteroarylalkylamino wie Chlorpyridylamino und Chlorpyridylmethylamino.

A steht besonders bevorzugt für Wasserstoff sowie für gegebenenfalls substituierte Reste aus der Reihe Acyl, Alkyl, Aryl, die bevorzugt die bei R angegebenen Bedeutungen haben. A steht ferner für eine bifunktionelle Gruppe. Genannt sei gegebenenfalls substituiertes Alkylen mit 1-4, insbesondere 1-2 C-Atomen, wobei als Substituenten die weiter oben aufgezählten Substituenten genannt seien und wobei die Alkylengruppen durch Heteroatome aus der Reihe N, O, S unterbrochen sein können.

A und Z können gemeinsam mit den Atomen, an welche sie gebunden sind, einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei Alkyl der N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.
Als Beispiele für den heterocyclischen Ring seien Pyrrolidin, Piperidin, Piperazin, Hexamethylenimin, Hexahydro-1,3,5-triazin, Morpholin genannt, die gegebenenfalls bevorzugt durch Methyl substituiert sein können.

E steht für einen elektronentziehenden Rest, wobei insbesondere $NO_2$, CN, Halogenalkylcarbonyl wie 1,5-Halogen-$C_{1-4}$-carbonyl, insbesondere $COCF_3$ genannt seien.

X steht für -CH= oder -N=

Z steht für gegebenenfalls substituierte Reste Alkyl, -OR, -SR, -NRR, wobei R und die Substituenten bevorzugt die oben angegebene Bedeutung haben.

Z kann außer dem obengenannten Ring gemeinsam mit dem Atom, an welches es gebunden ist und dem Rest

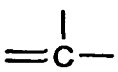

an der Stelle von X einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei die Alkyl oder N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.
Als Beispiele für den heterocyclischen Ring seien Pyrrolidin, Piperidin, Piperazin, Hexamethylenimin, Morpholin und N-Methylpiperazin genannt.

Als ganz besonders bevorzugt erfindungsgemäß verwendbare Verbindungen seien Verbindungen der allgemeinen Formeln (II) und (III) genannt:

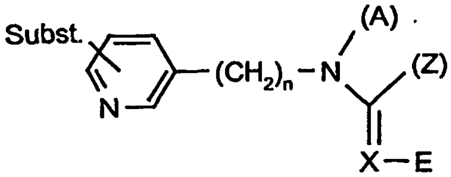

(II),

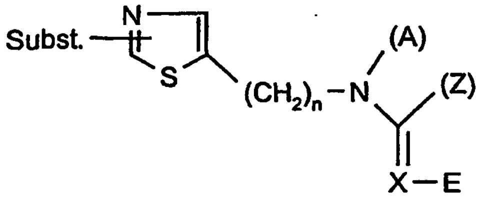 (III),

in welchen

n für 1 oder 2 steht,

Subst. für einen der oben aufgeführten Substituenten, insbesonders für Halogen, ganz besonders für Chlor, steht,
A, Z, X und E die oben angegebenen Bedeutungen haben,
Im einzelnen seien folgende besonders bevorzugte Verbindungen (Imidacloprid und Analoga) genannt:

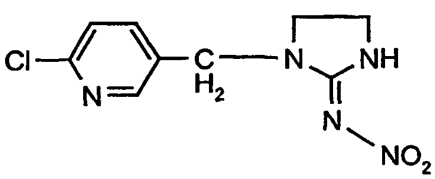

Imidacloprid

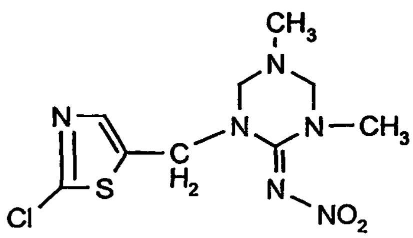

AKD 1022

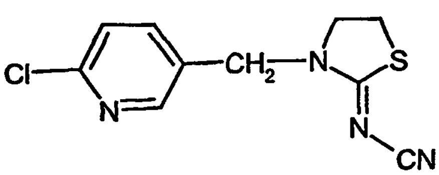

Thiacloprid

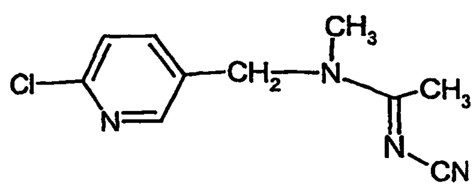

Acetamiprid

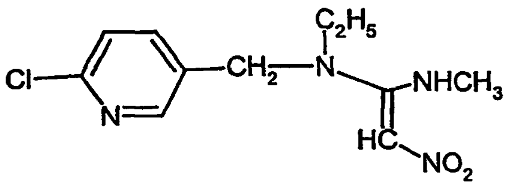

Ti 304

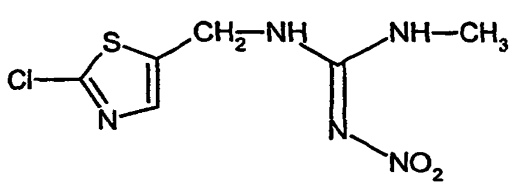

Ti 435

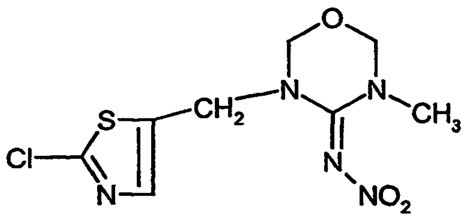

Thiamethoxam

Die N-Methylpyrrolidon-Menge kann im Bereich 27,5 bis 62,5 Gew.-% variiert werden. Bevorzugt liegt sie bei 35 bis 50 Gew.-%, besonders bevorzugt 40 bis 45 Gew.-%.

Die Antioxidantien-Mengen können im Bereich 0 - 0,5 % breit variiert werden, wobei Mengen im Bereich 0.05 - 0.25 % zu bevorzugen sind. Mengen im Bereich 0.05 -0.15 % werden zur Herstellung der erfindungsgemäßen Mittel besonders bevorzugt eingesetzt. Es kommen alle üblichen Antioxidantien in Frage, bevorzugt phenolische Antioxidantien wie z.B. Butylhydroxytoluol, Butylhydroxyanisol, Tocopherol.

Die Menge an organischer Säure kann im Bereich 0 - 0,5 % breit variiert werden, wobei Mengen im Bereich 0,05 - 0,25 % zu bevorzugen sind. Mengen im Bereich 0.05 -0.15 % werden zur Herstellung der erfindungsgemäßen Mittel besonders bevorzugt eingesetzt. Zum Einsatz in den erfindungsgemäßen Mitteln eignen sich alle pharmazeutisch verträglichen organischen Säuren, insbesondere Carbonsäuren, wie z.B. Citronensäure, Weinsäure, Milchsäure, Bernsteinsäure und Apfelsäure. Besonders bevorzugt sind die organischen Säuren Citronensäure und Apfelsäure. Ganz besonders bevorzugt ist Citronensäure. Ihre Menge kann insbesondere im Bereich 0,05 bis 0,25 breit variiert werden. Wobei die Mengen im Bereich 0,075 - 0,15 % wiederum besonders bevorzugt werden.

Die Co-Lösungsmittel-Mengen können im Bereich 2,5 - 10 Gew.% breit variiert werden, wobei Mengen im Bereich 2,5 - 7,5 Gew.-% zu bevorzugen sind. Mengen im Bereich 3,5 - 6,0 Gew.-% werden in den erfindungsgemäßen Mitteln besonders bevorzugt eingesetzt.

Als Co-Lösungsmittel kommen organische Lösungsmittel mit einem Siedepunkt >80°C und einem Flammenpunkt >75°C in Betracht. Bevorzugt haben die Co-Lösungsmitteln eine spreitende Wirkung. In diesem Zusammenhang sei auf höhersiedende aliphatische sowie aromatische Alkohole, aliphatische Polyether, aliphatische und/oder aromatische Ester, cyclische und/oder acyclische Carbonate hingewiesen.

Zur Herstellung der erfindungsgemäßen Mittel werden jedoch vorzugsweise aliphatische acyclische oder cyclische Ether bzw. Polyether sowie Fettsäureester insbesondere Triglyceride eingesetzt.

Zum Einsatz in den erfindungsgemäßen Mitteln eignen sich Ether bzw. Polyether beispielsweise aus der Reihe Diethylenglykolmonoethylther, Dipropylenglykolmonomethylether, Tetrahydrofurfurylalkohol und Tetrahydrofurfurylethoxylat, wobei die beiden letztgenannten besonders zu bevorzugen sind.

Als Fettsäureester sowie Triglyceride seien beispielsweise erwähnt: Isopropylmyristat, Miglyol 810, Miglyol 812, Miglyol 818, Miglyol 829, Miglyol 840 und Miglyol 8810 (zur Definition der Miglyole siehe beispielsweise H.P. Fiedler Lexikon der Hilfsstoffe für Pharmazie, Kosmetik und angrenzende Gebiete, Seiten 1008-1009, Bd. 2, Edito Cantor Verlag Aulendorf (1996)).

Den bisherigen Untersuchungen kann entnommen werden, dass die mit den genannten Co-Lösungsmitteln modifizierten, erfindungsgemäßen Mischungen sich durch ihre bessere Haut- und Augenverträglichkeit, bessere biologische Wirksamkeit sowie durch ihr günstigeres Kältestabilitätsverhalten in den üblichen Single-dose-Applikationstuben auszeichnen.

Neben den oben aufgeführten Bestandteilen können die erfindungsgemäße Mittel weitere übliche, pharmazeutisch annehmbare Hilfsstoffe enthalten. Als solche seien beispielsweise genannt: Spreitmittel und Tenside.

Spreitmittel sind beispielsweise spreitende Öle wie Adipinsäure-di-2-ethylhexylester, Isopropylmyristat, Dipropylenglykolpelargonat, cyclische und acyclische Silikonöle, wie Dimetikone und ferner deren Co- und Terpolymerisate mit Ethylenoxid, Propylenoxid und Formalin, Fettsäureester, Triglyceride, Fettalkohole.

Als Tenside seien genannt: Nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Die erfindungsgemäßen Mittel können nach üblichen Verfahren hergestellt werden, beispielsweise in dem man die Wirkstoffe unter Rühren mit den weiteren Bestandteilen vermischt und eine Lösung herstellt. Diese kann

gegebenenfalls filtriert werden. Zur Abfüllung eignen sich beispielsweise Kunststofftuben.

Die erfindungsgemäßen Flüssigformulierungen zeichnen sich durch ihre hervorragende Lagerungsstabilität von mindestens drei Jahren in allen Klimazonen aus. Aufgrund der sehr guten Wirksamkeit kann das Applikationsvolumen klein gehalten werden. Die bevorzugten Applikationsvolumina liegen bei 0,075-0,25 ml/1,0 kg [Körpergewicht des zu behandelnden Kleintieres], vorzugsweise 0,1-0,15 ml/1,0 kg [Körpergewicht des zu behandelnden Kleintieres].

Sie eignen sich hervorragend zum Abfüllen und Ausbieten in lagerungskritischen Behältern, wie z.B. der "Single dose Polypropylenkunststofftuben" der Wandstärke von 300-500 µm und des Abfüllvolumens 1,0 bis 4,0 ml.

Die erfindungsgemäßen Mittel sind weiterhin ausgezeichnet hautverträglich und weisen eine geringe Toxizität auf.

Schließlich sind sie aufgrund ihrer biologischen Abbaubarkeit umweltverträglich.

## Beispiele

### Beispiel 1

Eine homogene Spot-on-Lösung bestehend aus

45 g Permethrin mit 40 % cis und 60 % trans-Isomerenanteil
10 g Imidacloprid (1-[(6-Chlor-3-pyridinyl)methyl]-N-nitro-2-imidazolidinium) der Fa. Bayer AG
44,8 g N-Methylpyrrolidon
0,1 g Citronensäure
0,1 g BHT (Butylhydroxytoluol)

### Beispiel 2

Eine homogene Spot on Lösung bestehend aus

45 g Permethrin mit 40 % cis und 60 % trans-Isomerenanteil
10 g Imidacloprid
40,8 g N-Methylpyrrolidon
4,0 g Wasser
0,1 g Citronensäure
0,1 g BHT

### Beispiel 3

Eine homogene Spot on Lösung bestehend aus

45 g Permethrin mit 40 % cis und 60 % trans-Isomerenanteil
10 g Ti 435, Chlothianidine Fa. Takeda AG
44,8 g N-Methylpyrrolidon
0,1 g Citronensäure
0,1 g BHT

### Beispiel 4

Eine homogene Spot on Lösung bestehend aus

45 g Permethrin mit 40 % cis und 60 % trans-Isomerenanteil
10 g Diacloden (Thiamethoxam) der Fa. Novartis AG
44,8 g N-Methylpyrrolidon
0,1 g Citronensäure
0,1 gBHT

### Beispiel 5

Eine homogene Spot on Lösung bestehend aus

45 g Permethrin mit 40 % cis und 60 % trans-Isomerenanteil
7,5 g Imidacloprid
43,3 g N-Methylpyrrolidon
4,0 g Wasser
0,1 g Citronensäure
0,1 g BHT

**Beispiel 6**

Eine homogene Spot on Lösung bestehend aus

47,5 g Permethrin mit 40 % cis und 60 % trans-Isomerenanteil 10,0 g Imidacloprid
38,3 g N-Methylpyrrolidon
4,0 g Wasser
0,1 g Citronensäure
0,1 g BHT (Butylhydroxytoluol)

**Beispiel 7**

Eine homogene Spot on Lösung bestehend aus

47,5 g Permethrin mit 40 % cis und 60 % trans-Isomerenanteil
10 g Imidacloprid
42,3 g N-Methylpyrrolidon
0,1 g Citronensäure
0,1 g BHT

**Beispiel 8**

Eine homogene Spot on Lösung bestehend aus

45 g Permethrin mit 40 % cis und 60 % trans-Isomerenanteil
8 g Imidacloprid
46,8 g N-Methylpyrrolidon
0,1 g Milchsäure
0,1 g BHT

**Beispiel 9**

Eine homogene Spot on Lösung bestehend aus

45 g Permethrin mit 40 % cis und 60 % trans-Isomerenanteil
8 g Imidacloprid
46,8 g N-Methylpyrrolidon
0,1 g Milchsäure
0,1 g Butylhydroxyanisol

**Beispiel 10**

Eine homogene Spot-on-Lösung bestehend aus

45 g Permethrin mit 40 % cis und 60 % trans-Isomerenanteil
10 g Imidacloprid (1-[(6-Chlor-3-pyridinyl)methyl]-N-nitro-2-imidazolidinium) der Fa. Bayer AG
39,8 g N-Methylpyrrolidon
0,1 g Citronensäure
0,1 g BHT (Butylhydroxytoluol)
5,0 g Miglyol 812 der Fa. Sasol Germany GmbH, D- 58453 Witten

**Beispiel 11**

Eine homogene Spot on Lösung bestehend aus

45 g Permethrin mit 40 % cis und 60 % trans-Isomerenanteil
10 g Imidacloprid
35,8 g N-Methylpyrrolidon
4,0 g Wasser
0,1 g Citronensäure
0,1 g BHT
5,0 g Miglyol 840 der Fa. Sasol Germany GmbH, D- 58453 Witten

**Beispiel 12**

Eine homogene Spot on Lösung bestehend aus

45 g Permethrin mit 40 % cis und 60 % trans-Isomerenanteil
10 g Ti 435, Chlothianidine Fa. Takeda AG
39,8 g N-Methylpyrrolidon
0,1 g Citronensäure
0,1 g BHT
5,0 g Tetrahydrofurfurylalkohol

**Beispiel 13**

Eine homogene Spot on Lösung bestehend aus

45 g Permethrin mit 40 % cis und 60 % trans-Isomerenanteil
10 g Diacloden (Thiamethoxam) der Fa. Novartis AG
39,8 g N-Methylpyrrolidon
0,1 g Citronensäure
0,1 g BHT
5,0 g Tetrahydrofurfurylethoxylat

**Beispiel 14**

Eine homogene Spot on Lösung bestehend aus

45 g Permethrin mit 40 % cis und 60 % trans-Isomerenanteil
7,5 g Imidacloprid
40,0 g N-Methylpyrrolidon
0,1 g Citronensäure
0,1 g BHT
3,3 g Miglyol 812

**Beispiel 15**

Eine homogene Spot on Lösung bestehend aus

47,5 g Permethrin mit 40 % cis und 60 % trans-Isomerenanteil
10,0 g Imidacloprid
33,8 g N-Methylpyrrolidon
4,0 g Wasser
0,1 g Citronensäure
0,1 g BHT (Butylhydroxytoluol)
5,0 g Miglyol 812

**Beispiel 16**

Eine homogene Spot on Lösung bestehend aus

47,5 g Permethrin mit 40 % cis und 60 % trans-Isomerenanteil
10 g Imidacloprid
34,3 g N-Methylpyrrolidon
0,1 g Citronensäure
0,1 g BHT
4,0 g Tetrahydrofurfurylalkohol
4,0 g Miglyol 812

**Beispiel 17**

Eine homogene Spot on Lösung bestehend aus

45 g Permethrin mit 40 % cis und 60 % trans-Isomerenanteil
8 g Imidacloprid
40,8 g N-Methylpyrrolidon
0,1 g Milchsäure
0,1 g BHT
6,0 g Tetrahydrofurfurylakohol

**Beispiel 18**

Eine homogene Spot on Lösung bestehend aus

45 g Permethrin mit 40 % cis und 60 % trans-Isomerenanteil
8 g Imidacloprid
42,8 g N-Methylpyrrolidon
0,1 g Milchsäure
0,1 g Butylhydroxyanisol
4,0 g Diethylenglykolmonoethylether

**A. Wirksamkeit gegen Flöhe am Hund**

*Ctenocephalides felis*

An den Tagen -4 und -1 werden Hunde mit ca. 100 adulten, nüchternen *Ctenocephalides felis* pro Hund infestiert. Dabei werden die Flöhe auf den Nacken des Tieres ausgebracht.

Am Tag 0 wird der Infestationserfolg am Hund überprüft, indem am wachen Tier nach Flöhen gesucht wird. Die Zahl der lebenden Flöhe wird protokolliert.

Nach der Zählung der Flöhe werden die Tiere behandelt. Die Hunde der Kontrollgruppe werden nicht behandelt. Die zu prüfenden Arzneimittel gemäß der Beispiele 1 bis 18 werden den Tieren dermal als Spot-on bei einer Applikationsmenge von 0,1 ml/kg Körpergewicht verabreicht. Die Applikation erfolgt einmalig am Tag 0. Es werden nur klinisch gesunde Tiere verwendet.

Am Tag 1 werden alle Hunde auf lebende Flöhe überprüft. Die Ergebnisse werden in den Rohdaten festgehalten.

Am Tag 7, 14, 21 und 28 werden alle Hunde mit ca. 100 adulten, nüchternen *Ctenocephalides felis* pro Hund reinfestiert. Jeweils einen Tag nach Reinfestation werden alle Hunde auf lebende Flöhe kontrolliert. Die Ergebnisse werden in den Rohdaten protokolliert.

Eine Formulierung wird als hochwirksam erachtet, wenn am Tag 1 und jeweils am zweiten Tag nach Reinfestation eine Wirksamkeit >95% festgestellt wird und diese Wirkung über mindestens 3-4 Wochen anhält.

Für die Berechnung der Wirksamkeit wird eine modifizierte Formel nach Abbott benutzt:

$$\text{Wirksamkeit \%} = \frac{\text{Ø Anzahl Flöhe KG - Ø Anzahl Flöhe BG}}{\text{Ø Anzahl Flöhe KG}} \text{ X } 100$$

KG: Kontrollgruppe
BG: Behandlungsgruppe

Die Arzneimittel gemäß den Formulierungsbeispielen 1 bis 18 in einer Dosierung von 0,1ml/kg als Spot on appliziert, erwiesen sich gegen *Ctenocephalides felis* als hochwirksam.

**B. Wirksamkeit gegen Zecken (*Rhipicefalus sanguineus*) am Hund**

Jeweils an den Tagen -4 und -1 werden Hunde mit 2% Rompun® (Bayer AG, Wirkstoff: Xylazinhydrochlorid) (0,1ml/kg Körpergewicht) sediert. Nachdem alle Hunde sediert sind (nach ca. 10-15 Minuten) werden sie in Transportboxen überführt und 50 *Rhipicefalus sanguineus* (25♀, 25♂) pro Hund auf den Nacken des Tieres ausgebracht. Die Tiere werden nach ca. 1 ½ Stunden wieder aus der Transportkiste in den Käfig gesetzt.

Am Tag 0 wird der Infestationserfolg am Hund überprüft, indem am wachen Tier nach Zecken gesucht wird. Intensiv wird dabei gesucht im Kopf- und Ohrenbereich inkl. Ohrenfalte, im Bereich des Nackens, am Unterbauch, an der Unterbrust, an der seitlichen Flanke sowie zwischen den Zehen und an den Gliedmaßen. Die Zahl der angesogenen lebenden Zecken wird protokolliert. Tote Zecken werden entfernt.

Nach der Zählung der Zecken werden die Tiere behandelt. Die Hunde der Kontrollgruppe werden nicht behandelt. Die zu prüfenden Arzneimittel werden den Tieren dermal als Spot-on verabreicht. Die Applikation erfolgt einmalig am Tag 0. Es werden nur klinisch gesunde Tiere verwendet.

Am Tag 1 und Tag 2 werden alle Hunde auf lebende und tote angesogende Zecken überprüft. Die Ergebnisse werden in den Rohdaten festgehalten. Am Tag 2 werden alle lebenden und toten Zecken vom Hund entfernt.

Am Tag 7, 14, 21 und 28 werden alle Hunde mit jeweils 50 *Rhipicefalus sanguineus* (25♀, 25♂) pro Hund reinfestiert. Jeweils einen und zwei Tage nach Reinfestation werden alle Hunde auf lebende und tote angesogene Zecken kontrolliert. Die Ergebnisse werden in den Rohdaten protokolliert. Am zweiten Tag nach Reinfestation werden alle lebenden und toten Zecken vom Hund entfernt.

Eine Formulierung wird als hochwirksam erachtet, wenn am Tag 2 und jeweils am zweiten Tag nach Reinfestation eine Wirksamkeit >90 % festgestellt wird und diese Wirkung über mindestens 3 Wochen anhält.

Für die Berechnung der Wirksamkeit wird eine modifizierte Formel nach Abbott benutzt:

$$\text{Wirksamkeit \%} = \frac{\text{Ø Anzahl Zecken KG - Ø Anzahl Zecken BG}}{\text{Ø Anzahl Zecken KG}} \text{ X } 100$$

KG: Kontrollgruppe
BG: Behandlungsgruppe

Die Arzneimittel in einer Dosierung gemäß den Formulierungsbeispielen 1 bis 18 von 0,1ml/kg als Spot on appliziert, erwiesen sich gegen *Rhipicefalus sanguineus* als hochwirksam.

**C. Floh- und Zeckenwirksamkeit über 6 Wochen**

Die Floh- und Zeckenwirksamkeit der erfindungsgemäßen Mittel wurde über 6 Wochen getestet. Die Versuchsdurchführung folgte der Beschreibung unter den Punkten A und B

**Tabelle 1** Floh- und Zeckenwirksamkeit des Mittels gemäß Beispiel 10

| Studien-nummer | Studien-Design/Applikations-volumen 0,1 ml/kg | Floh-Wirksam-keit (geo Mean)/-Zecken-Wirk-samkeit (geo. mean) 1-2 Tage nach der Behand-lung | Floh-Wirksam-keit (geo Mean)/-Zecken-Wirk-samkeit (geo. mean) 1 Woche nach der Behand-lung | Floh-Wirksam-keit (geo Mean)/-Zecken-Wirk-samkeit (geo. mean) 2 Wochen nach der Behand-lung | Floh-Wirksam-keit (geo Mean)/-Zecken-Wirk-samkeit (geo. mean) 3 Wochen nach der Behand-lung | Floh-Wirksam-keit (geo Mean)/-Zecken-Wirk-samkeit (geo. mean) 4 Wochen nach der Behand-lung | Floh-Wirksam-keit (geo Mean)/-Zecken-Wirk-samkeit (geo. mean) 5 Wochen nach der Behand-lung | Floh-Wirksam-keit (geo Mean)/Zecken-Wirksamkeit (geo. mean) 6 Wochen nach der Behand-lung |
|---|---|---|---|---|---|---|---|---|
| 1 | Ctenocephalides felis / Rhipicephalus sanguineus | 100% / 87,2% | 100% / 89,9% | 100% /89,9% | 95,3% / 97,6% | 95,9% /91,4% | 90,6% / 85,5% | 92,3% /83,6% |
| 2 | Ctenocephalides felis / Amblyomma americanum | 100% / 38,9% | 100% / 100% | 100% / 100% | 99,7% / 100% | 99,0% / 50,0% | 96,3% / 92,8% | 99,0% / 50,0% |
| 3 | Ctenocephalides felis / Rhipicephalus sanguineus | 100% / 67,0% | 100% / 95,9% | 99,8% / 96,8% | 98,9% / 94,1 % | 94,5% / 85,0% | 68,1% / 80,0% | |

**Patentansprüche**

1. Mittel enthaltend,

a) 35 - 60 Gew.-% des Wirkstoffs Permethrin
b) 2.5 - 12.5 Gew.-% Imidacloprid oder Imidaclopridanalogon der Formel (I):

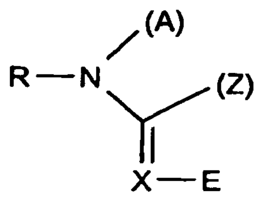

(I),

in welcher

R für Wasserstoff, gegebenenfalls substituierte Reste der Gruppe Acyl, Alkyl, Aryl, Aralkyl, Heteroaryl oder Heteroarylalkyl steht;

A für eine monofunktionelle Gruppe aus der Reihe Wasserstoff, Acyl, Alkyl, Aryl steht oder für eine bifunktionelle Gruppe steht, die mit dem Rest Z verknüpft ist;

E für einen elektronenziehenden Rest steht;

X für die Reste -CH= oder =N- steht, wobei der Rest -CH= anstelle eines H-Atoms mit dem Rest Z verknüpft sein kann;

Z für eine monofunktionelle Gruppe aus der Reihe Alkyl, -O-R, -S-R,

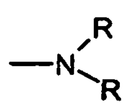

steht, oder für eine bifunktionelle Gruppe steht, die mit dem Rest A oder dem Rest X verknüpft ist, wobei die Substituenten der oben aufgeführten gegebenenfalls substituierten Reste ausgewählt sind aus: Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen, wobei die Halogenatome gleich oder verschieden sind; Hydroxy; Halogen; Cyano; Nitro; Amino; Monoalkyl- und Dialkylamino mit 1 bis 4 Kohlenstoffatomen, Carboxyl; Carbalkoxy mit 2 bis 4; Sulfo ($-SO_3H$); Alkylsulfonyl mit 1 bis 4 Kohlenstoffatomen; Arylsulfonyl mit 6 oder 10 Arylkohlenstoffatomen sowie Heteroarylamino und Heteroarylalkylamino;

c) 27.5 - 62.5 Gew.-% N-Methylpyrrolidon
d) 0 - 5 Gew.-% Wasser
e) 0 - 0,5 Gew.-% phenolische Antioxidantien und
f) 0 - 0,5 Gew.-% organische Säuren.

2. Mittel gemäß Anspruch 1, enthaltend als Komponente b) 2.5 - 12.5 Gew.-% Imidacloprid oder Imidaclopridanalogon der Formeln (II) oder (III):

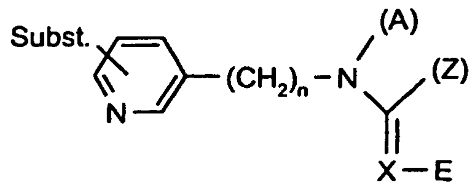

(II),

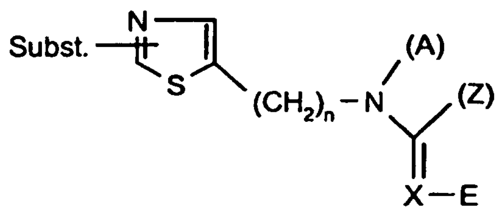

(III),

in welchen

n für 1 oder 2 steht,
Subst. für einen der in Anspruch 1 aufgeführten Substituenten, insbesondere für Halogen steht,
A, Z, X und E die in Anspruch 1 angegebenen Bedeutungen haben.

**3.** Mittel gemäß Anspruch 1, enthaltend als Komponente b) 2.5 - 12.5 Gew.-% Imidacloprid oder eine Imidaclopridanalogon, ausgewählt aus:

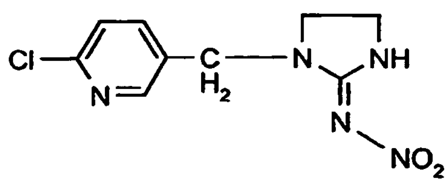

**Imidacloprid**

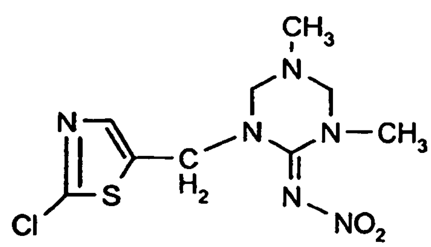

**AKD 1022**

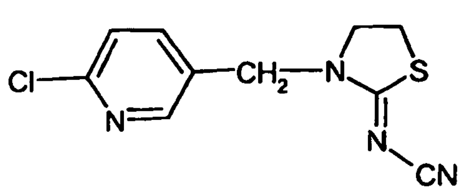

Thiacloprid

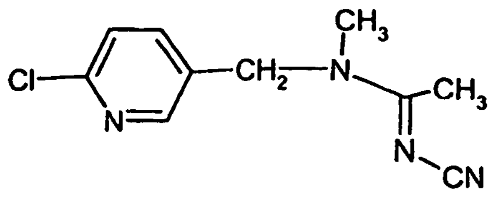

Acetamiprid

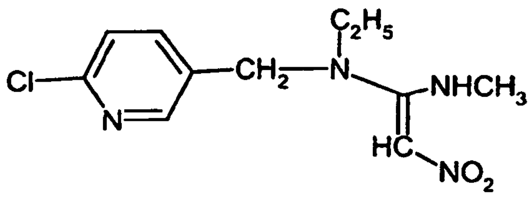

Ti 304

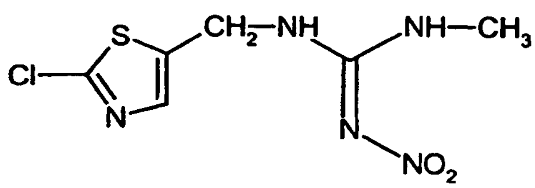

Ti 435

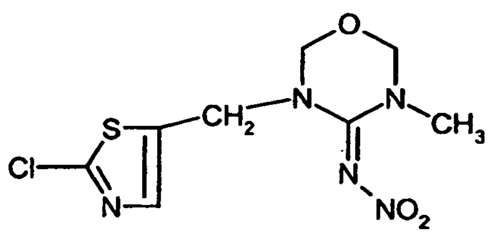

Thiamethoxam

**4.** Mittel gemäß einem der vorstehenden Ansprüche, zusätzlich enthaltend

g) 2,5 - 10 Gew.-% Co-Lösungsmittel.

**5.** Verfahren zur Herstellung von Mitteln gemäß einem der vorstehenden Ansprüche, **dadurch gekennzeichnet, dass** man Permethrin und Imidacloprid oder Imidacloprid-Analogon gemäß einem der Ansprüche 1 bis 4 mit den übrigen in den Ansprüchen 1 bis 4 genannten Bestandteilen unter Rühren vermischt und eine Lösung herstellt.

**6.** Verwendung des Mittels gemäß einem der Ansprüche 1 bis 4 zur Herstellung von Arzneimitteln zur Bekämpfung von Parasiten an Tieren.

**7.** Verwendung gemäß Anspruch 6, zur Bekämpfung von Zecken und/oder Flöhen an warmblütigen Tieren.

**8.** Verwendung gemäß einem der Ansprüche 6 oder 7, zur Bekämpfung von Zecken und/oder Flöhen an Hunden.

**9.** Verwendung gemäß einem der Ansprüche 6 oder 7, zur Bekämpfung von Zecken und/oder Flöhen an Katzen.

## Claims

1. Compositions, comprising

a) 35 - 60% by weight of the active compound permethrin
b) 2.5 - 12.5% by weight of imidacloprid or an imidacloprid analogue of the formula (I):

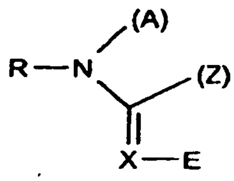

(I),

in which

R represents hydrogen, optionally substituted radicals of the group consisting of acyl, alkyl, aryl, aralkyl, heteroaryl and heteroarylalkyl;

A represents a monofunctional group from the group consisting of hydrogen, acyl, alkyl, aryl, or represents a bifunctional group which is attached to the radical Z;

E represents an electron-withdrawing radical;

X represents the radicals -CH= or =N-, where the radical -CH= may be attached to the radical Z instead of an H-atom;

Z represents a monofunctional group from the group consisting of alkyl, -O-R, -S-R,

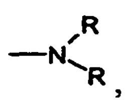

or represents a bifunctional group which is attached to the radical A or the radical X,
where the substituents of the optionally substituted radicals listed above are selected from the group consisting of: alkyl having 1 to 4 carbon atoms, alkoxy having 1 to 4 carbon atoms, alkylthio having 1 to 4 carbon atoms, haloalkyl having 1 to 4 carbon atoms and 1 to 5 halogen atoms, where the halogen atoms are identical or different; hydroxyl; halogen; cyano; nitro; amino; monoalkyl- and dialkylamino having 1 to 4 carbon atoms; carboxyl; carbalkoxy having 2 to 4; sulfo ($-SO_3H$); alkylsulphonyl having 1 to 4 carbon atoms; arylsulphonyl having 6 or 10 aryl carbon atoms and also heteroarylamino and heteroarylalkylamino;
c) 27.5 - 62.5% by weight of N-methylpyrrolidone
d) 0 - 5% by weight of water
e) 0 - 0.5% by weight of phenolic antioxidants and
f) 0 - 0.5% by weight of organic acids.

2. Composition according to claim 1, comprising, as component b), 2.5-12.5% by weight of imidacloprid or imidacloprid analogue of the formula (II) or (III):

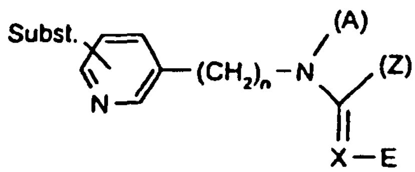

(II),

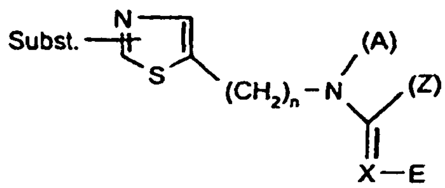

(III),

in which

n represents 1 or 2,
Subst. represents one of the substituents listed in Claim 1, in particular halogen,
A, Z, X and E are as defined in Claim 1.

**3.** Composition according to Claim 1, comprising, as component b), 2.5-12.5% by weight of imidacloprid or an imidacloprid analogue selected from the group consisting of:

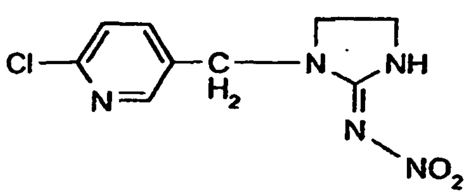

imidacloprid

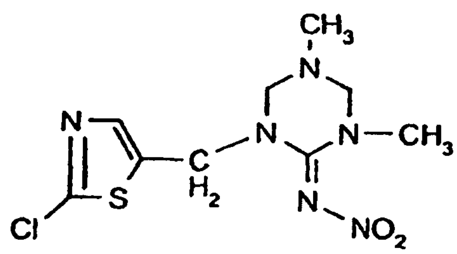

AKD 1022

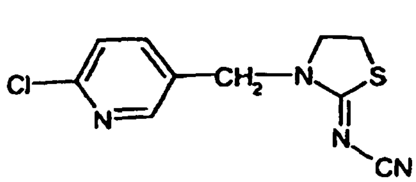

thiacloprid

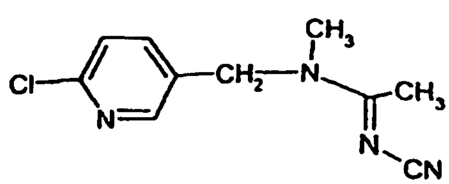

acetamiprid

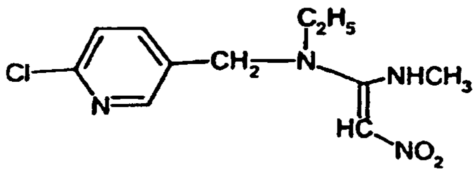

Ti 304

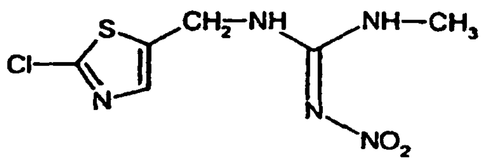

Ti 435

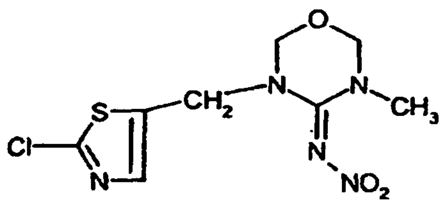

thiamethoxam

4. Composition according to any of the preceding claims, additionally comprising

   g) 2.5-10% by weight of cosolvent.

5. Process for preparing compositions according to any of the preceding claims, **characterized in that** permethrin and imidacloprid or imidacloprid analogue according to any of Claims 1 to 4 are mixed, with stirring, with the other components mentioned in Claims 1 to 4, and a solution is prepared.

6. Use of the composition according to any of Claims 1 to 4 for preparing medicaments for controlling parasites on animals.

7. Use according to Claim 6 for controlling ticks and/or fleas on warm-blooded animals.

8. Use according to Claim 6 or 7 for controlling ticks and/or fleas on dogs.

9. Use according to Claim 6 or 7 for controlling ticks and/or fleas on cats.

**Revendications**

1. Agent contenant

   a) 35 à 60 % en poids de la substance active perméthrine
   b) 2,5 à 12,5 % en poids d'imidaclopride ou d'analogue de l'imidaclopride de la formule (I):

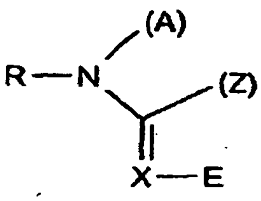

(I),

dans laquelle

R représente l'hydrogène, des radicaux, le cas échéant, substitués du groupe formé par l'acyle, l'alkyle, l'aryle, l'aralkyle, l'hétéroaryle ou l'hétéroarylalkyle;

A correspond à un groupe monofonctionnel appartenant à la série hydrogène, acyle, alkyle, aryle ou à un groupe bifonctionnel, qui est lié au radical Z;

E est égal à un radical capteur d'électrons;

X représente les radicaux -CH= ou =N-, le radical -CH= pouvant être lié avec le radical Z à la place d'un atome d'hydrogène;

Z correspond à un groupe monofonctionnel de la série alkyle, -O-R, -S-R,

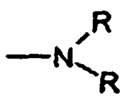

ou à un groupe bifonctionnel, qui est lié avec le radical A ou le radical X,
les substituants des radicaux, le cas échéant substitués, mentionnés ci-dessus étant sélectionnés parmi les suivants: alkyle avec 1 à 4 atomes de carbone, alcoxy avec 1 à 4 atomes de carbone, alkylthio avec 1 à 4 atomes de carbone, halogène-alkyle avec 1 à 4 atomes de carbone et 1 à 5 atomes d'halogène, les atomes d'halogène étant identiques ou différents; hydroxyle; halogène; cyano; nitro; amino; monoalkyl- et dialkylamino avec 1 à 4 atomes de carbone; carboxyle; carbalcoxy avec 2 à 4 atomes de carbone; sulfo ($-SO_3H$); alkylsulfonyle avec 1 à 4 atomes de carbone; arylsulfonyle avec 6 ou 10 atomes de carbone arylique, ainsi qu'hétéroarylamino et hétéroarylalkylamino;

c) 27,5 à 62,5 % en poids de N-méthylpyrrolidone
d) 0 à 5 % en poids d'eau
e) 0 à 0,5 % en poids d'antioxydants phénoliques et
f) 0 à 0,5 % en poids d'acides organiques.

**2.** Agent selon la revendication 1, contenant comme composant b), 2,5 à 12,5 % en poids d'imidaclopride ou d'analogue d'imidaclopride des formules (II) ou (III):

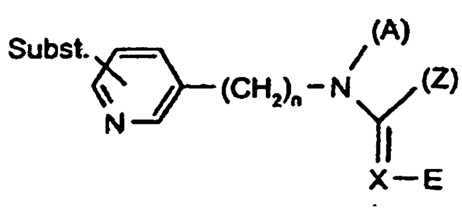

(II),

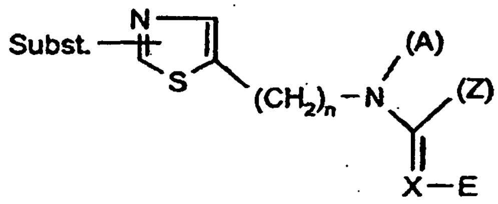

(III),

dans lesquelles

n représente 1 ou 2,
Subst. correspond à un des substituants mentionnés dans la revendication 1, en particulier un halogène,
A, Z, X et E possèdent la signification indiquée dans la revendication 1.

3. Agent selon la revendication 1, contenant comme composant b), 2,5 à 12,5 % en poids d'imidaclopride ou d'un analogue d'imidaclopride sélectionné parmi:

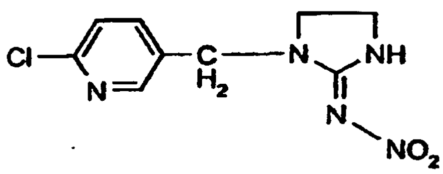

Imidacloprid

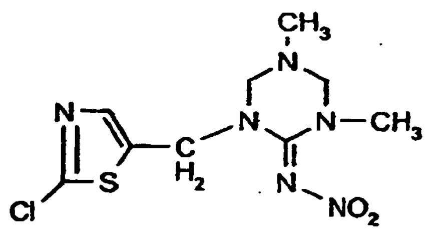

AKD 1022

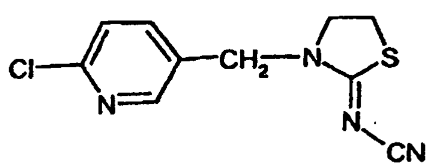

Thiacloprid

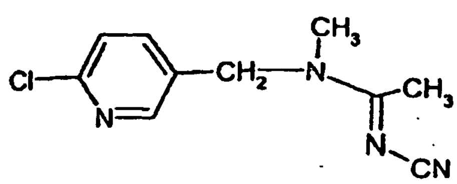

Acetamiprid

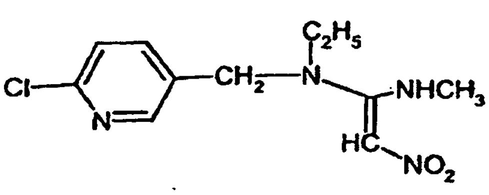

Ti 304

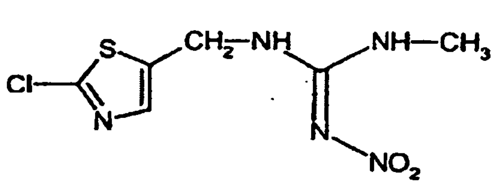

Ti 435

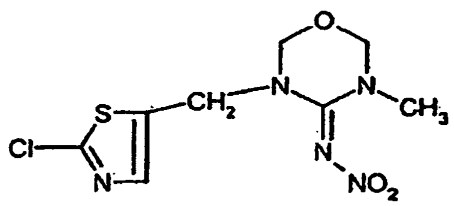

Thiamethoxam

4. Agent selon une des revendications qui précèdent, contenant en plus

g) 2,5 à 10 % en poids de cosolvant.

5. Procédé de préparation d'agents selon une des revendications qui précèdent, **caractérisé en ce que** l'on mélange sous agitation la perméthrine et l'imidaclopride ou l'analogue d'imidaclopride selon une des revendications 1 à 4 avec les autres constituants mentionnés dans les revendications 1 à 4, et que l'on prépare une solution.

6. Utilisation de l'agent selon une des revendications 1 à 4, pour préparer des médicaments destinés à combattre les parasites sur les animaux.

7. Utilisation selon la revendication 6, pour combattre les tiques et/ou les puces sur les animaux à sang chaud.

8. Utilisation selon une des revendications 6 ou 7, pour combattre les tiques et/ou les puces sur les chiens.

9. Utilisation selon une des revendications 6 ou 7, pour combattre les tiques et/ou les puces sur les chats.